# EUROPEAN PATENT SPECIFICATION

(11) **EP 0 590 721 B1**
(45) Date of publication and mention of the grant of the patent: **17.07.1996**
(21) Application number: 93202756.8
(22) Date of filing: 24.09.1993
(51) Int. Cl.: C12N 15/81, C12N 1/19, G01N 33/50, G01N 33/566, G01N 33/74

(54) **Method for expressing receptors of the human nervous system in the yeast Schizosaccharomyces pombe**
Verfahren zur Expression von Rezeptoren des menschlichen Nervensystems in der Hefe Schizosaccharomyces Pombe
Procédé d'expression de récepteurs du système nerveux humain chez la levure schizosacchoromyces pombe

(30) Priority: 29.09.1992 IT MI922243
(43) Date of publication of application: 06.04.1994
(73) Proprietor: ENICHEM S.p.A., I-20124 Milano (IT)
(72) Inventor: Ficca, Anna Grazia, I-00167 Rome (IT)
(74) Representative: Fusina, Gerolamo

(56) References cited:
- EP-A- 0 220 689
- EP-A- 0 475 304
- SCIENCE, vol. 250, 1990; K. KING et al., pp. 121-123
- THE EMBO JOURNAL, vol. 4, 1985; N. NAKAYAMA et al., pp. 2643-2648
- CELL, vol. 45, 1986; P. RUSSELL et al., pp. 145-153
- BIOLOGICAL CHEMISTRY, HOPPE-SEYLER, vol. 374, 1993; P. SANDER et al., p. 730

## Description

This invention relates to a method for expressing receptors of the human nervous system in cells of the eukaryotic micro-organism Schizosaccharomyces pombe.

More particularly the invention relates to a method for expressing receptors of the human nervous system in the cells of the eukaryotic micro-organism Schizosaccharomyces pombe by introducing a modified cloning vector into the micro-organism, and to the preparation of the cloning vector.

The present invention also provides a system which enables the interaction of new pharmaceutical products with human receptors to be quickly tested.

A micro-organism can be considered as a perfectly integrated machine which has evolved in the direction of its aim, namely survival and reproduction.

Because of the development of techniques which enable genes to be transferred from any source into a micro-organism, genetically programmed micro-organisms have been constructed able to produce substances absent at the level of their metabolism.

As is well known, deoxyribonucleonic acid (DNA), the genetic material, is a very thin long double helix, each strand of which is a chain the elements of which are known as a nucleotide. Each nucleotide consists of a phosphate group, a ribose molecule and one of four chemical groups known as bases, namely A (adenine); T (thymine); C (cytosine); G (guanine).

The sequence of these bases encodes the genetic information. In this respect, a gene is a DNA segment encoding the genetic information transcribed in messenger RNA (ribonucleonic acid) and then translated to form a protein.

In molecular cloning the DNA is bound to a molecule, generally a plasmid, which serves as a vector. The fused molecule obtained in this manner, known as recombinant DNA, is incubated together with bacterial or yeast cells to make them permeable. The transformed cells, ie those which receive the plasmid, are selected by various techniques and are cultivated in large quantity, either to produce a protein encoded by the inserted DNA fragment, or to study the structure and expression of the DNA fragment cloned in the plasmid.

Similar techniques have been applied to the microbiological synthesis of human insulin, the growth hormone and interferons.

Of yeasts, Saccharomyces cerevisiae has been best characterised genetically and chemically and most utilized in industrial microbiology. Together with the bacterium Escherichia coli it has become the micro-organism chosen as host for expressing cloned genes.

Many experts consider Saccharomyces cerevisiae to be the eukaryotic model for the study of the structure, the function and the regulation of the genes pertaining to higher organisms: "Escherichia coli in the eukaryotic world".

In eukaryotic organisms, proteins can undergo post-translational modifications, such as the addition of sugar (glycoprotein) chains, or phosphorylation (phosphoproteins).

The use of Saccharomyces cerevisiae for expressing heterologous proteins has frequently encountered great problems associated with the post-translational modification differences between this yeast and the higher eukaryotic organisms, in particular the human system.

In contrast, the yeast Schizosaccharomyces pombe has greater homologies with the higher eukaryotic systems, including with regard to protein post-translational modification mechanisms. However, excluding some exceptions as reported for example in Beclman R.B. and Gallalnder J.F. (1979): "Adv. Food. Res.", vol. 25, pp 1-52, the yeast Schizosaccharomyces pombe has not yet encountered much interest in the biotechnological industry. EP-A-220 689 discloses a vector for expressing proinsulin in Schizosaccharomyces pombe.

The present applicant has now found a method by which the micro-organism Schizosaccharomyces pombe is able to produce the protein of the β₂-adrenergic human membrane receptor, and by which it can be determined whether this protein preserves its pharmacological characteristics when produced in the yeast cell.

The present applicant also demonstrates that the yeast Schizosaccharomyces pombe is a versatile, easily manipulated micro-organism and can therefore be used as host for producing heterologous proteins, hence overcoming the drawbacks of the known art.

The yeast Schizosaccharomyces pombe offers the advantage of having proteins structurally and functionally similar to the proteins involved in the transmission of the nervous message of the higher organisms, as described for example in Kitamura K. and Shimoda C. (1991): "EMBO J."_{,} vol. 10, pp 3743-3749 and in Obara T. et al. (1991): "Proc. Natl. Acad. Sci.", USA vol. 88, pp 5877-5881.

The present invention therefore provides a method for expressing receptors of the human nervous system in the cells of the eukaryotic micro-organism Schizosaccharomyces pombe by the introduction of a modified cloning vector into the micro-organism, comprising:
a) constructing vectors for the cloning and expression of the human genes for the nervous system receptors;
b) inserting these vectors into a suitable yeast strain.

The human nervous system receptor used for the purposes of the present invention is the β₂ adrenergic human receptor, a membrane protein widespread in organs such as the brain, the lung and the placenta, and which is activated by the bond with the natural agonist adrenaline.

The vectors for cloning and expressing genes in the yeast strains are constructed using plasmids (circular DNA molecules).

The yeast plasmids present specific DNA sequences which control their replication (ARS and 2µ sequences) and allow them equal distribution during cell growth. These plasmids also contain the bacterial origins of replication (ORI) and can therefore replicate in Escherichia coli.

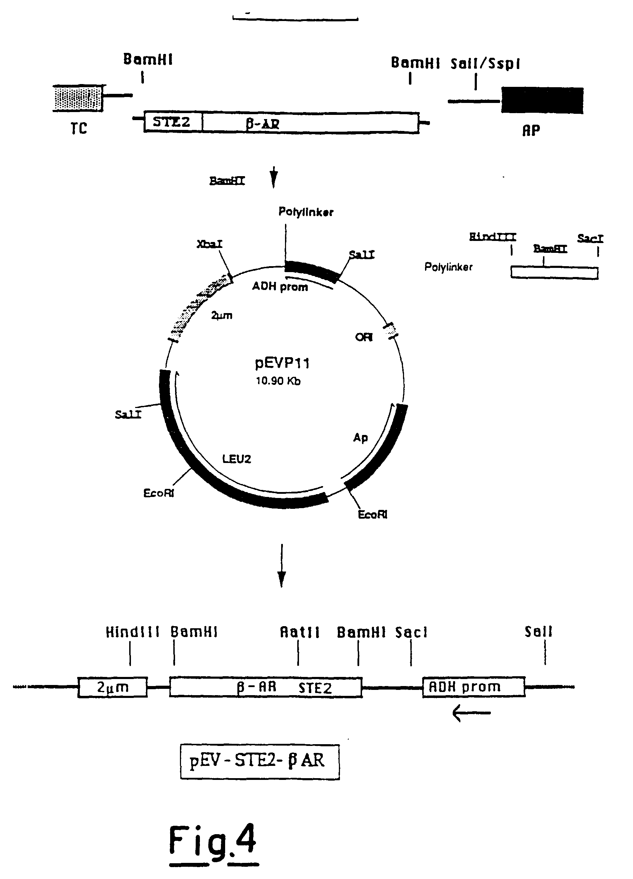

The yeast plasmid used for the purposes of the present invention is pEVP11 described by Russel P. and Nurse P. (1986): "Cell", vol. 45, pp 145-153 (Figure 4). It has a polylinker region consisting of a series of restriction sites useful for cloning. This region is preceded by a DNA segment essential for effective expression of the cloned gene under particular growth conditions, and corresponds to the constituent promoter of the encoding gene for alcohol dehydrogenase (adh).

The β₂-adrenergic gene is cloned in the yeast vector in the following manner.

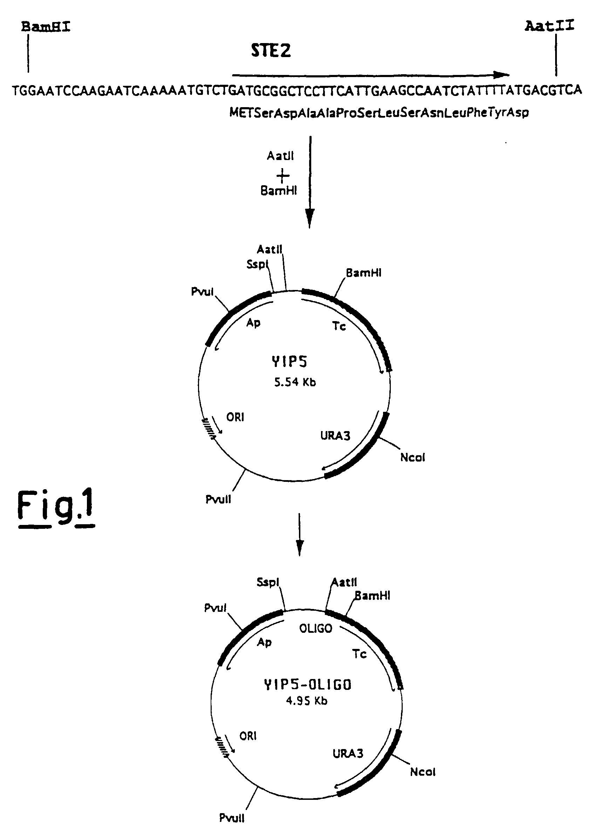

A double-strand oligonucleotide was synthesized with 64 nucleotide bases corresponding to the 11 non-encoding bases and the first 42 encoding bases of the gene STE2 of the yeast Saccharomyces cerevisiae, specific for the membrane receptor STE2 which has considerable homologies with the human adrenergic receptor [Nakayama N. et al. (1985): "EMBO J.", vol 4, pp 2643-2648, Figure 1].

Two restriction sites recognized by the enzymes BamHI and Aat-II were created at the end of the oligonucleotide.

After purification by acrylamide gel at 20% concentration, the oligonucleotide was cloned in the YIP5 yeast vector (Struhl K. et al,: "Proc. Natl. Acad. Sci.", USA, vol 76, pp 1035-1039) linearized with the restriction enzymes BamHI and Aat-II (Figure 1).

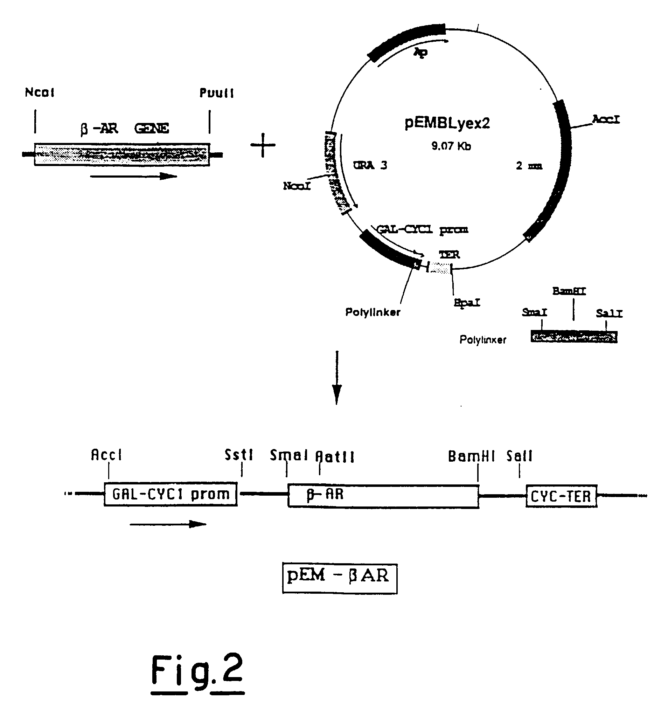

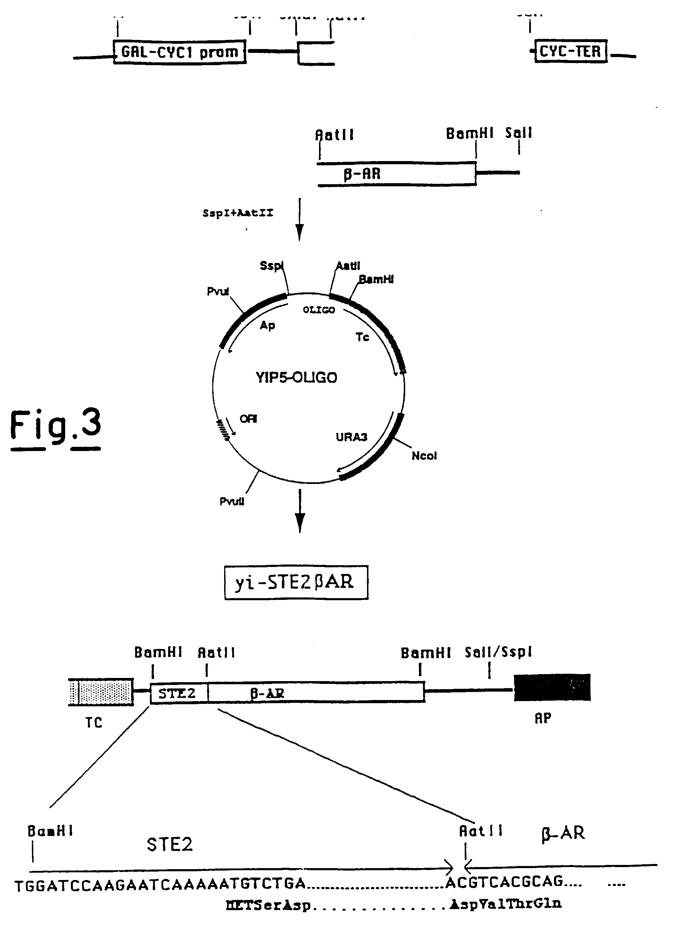

The 1.6 kb restriction fragment NcoI-PvuII of the β₂-adrenergic human gene was cloned in the SmaI site of the yeast plasmid pEMBLyex2 (Figure 2). The resultant plasmid pEM-β₂AR was linearized with the restriction enzyme SalI, the 5' ends being filled with deoxynucleotides by the Klenow enzyme, a second digestion then being effected with the enzyme Aat-II which recognizes the site GACGTC present in the β₂-adrenergic gene, to deprive this latter of the first 69 encoding bases. After eluting with agarose gel, the restriction fragment Aat-II-SalI (Figure 3) of the β₂-adrenergic gene was cloned in the plasmid YIP5-oligo, digested in its turn with the enzymes SspI and Aat-II.

The resultant plasmid, yi-STE2-β₂AR, contains that region of the yeast encoding the entire human receptor starting from the 23rd amino acid. In this respect, the deletion of the first 23 amino acids of the amino-terminal end of the gene β₂AR was replaced by the encoding sequence for the first 14 amino acids of the gene STE2 of Saccharomyces cerevisiae.

The plasmid yi-STE2-β₂AR is however unable to express the chimeric gene STE2-β₂AR as it does not possess a yeast promoter essential for controlling the expression of the heterologous gene in the yeast.

To express the chimeric protein the plasmid pEVP11 was chosen, in which the adh promoter of Schizosaccharomyces pombe is cloned.

The DNA sequence of the adh promoter constitutionally controls the expression of the cloned genes downstream of the promoter when the transformed yeast cells are made to grow in the presence of glucose.

The plasmid yi-STE2-β₂AR was digested with the enzyme BamHI and the DNA fragment containing the STE2-β₂AR chimeric gene sequence was isolated after passing through agarose gel and cloned in the BamHI site of the plasmid pEVP11.

The plasmid with the chimeric gene correctly orientated, pEV-STE2-β₂AR is shown in Figure 4.

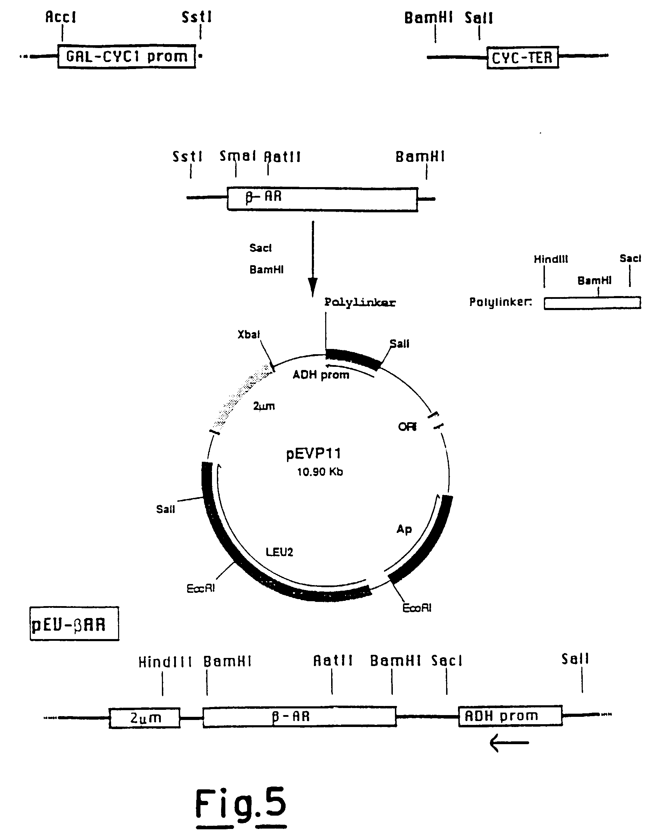

Figure 5 shows the cloning of the restriction fragment SstI-BamHI containing the encoding gene for the receptor β₂AR in the vector pEV11.

The Schizosaccharomyces pombe yeast strain used for the purposes of the present invention is an auxotrophic strain for the marker leu2, which is unable to grow in culture medium not containing the amino acid leucine.

In this respect, the plasmid pEV-STE2-β₂AR was used to transform the Schizosaccharomyces pombe yeast strain leul-32 h- using the method described by Klebe et al. (1983): "GENE", vol. 25, pp 333-341.

The yeast transformants containing the plasmid were selected for their ability to grow at 30°C for 6-10 days on plates containing EMM culture medium described by Burse P. (1975): "Nature", vol 256, page 574, containing 1.2% of sorbitol, free of the amino acid leucine.

The thus selected Schizosaccharomyces pombe transformants were transferred to EMM liquid culture medium containing 2% of glucose and 40µM of alprenolol, and left to grow at 30°C under continuous agitation.

After about 36 hours of growth, the cultures had reached a density of A₆₀₀ₙₘ = 3 O.D., corresponding to a concentration of 6x10⁷ cells/ml.

The cells were centrifuged at 5000 rpm, re-suspended in 6 ml of SCE buffer described in Table 1, plus 0.05 mg/ml of the enzyme Zymolase produced by KIRON. After incubation at 37°C for 30 minutes, the spheroplasts obtained in this manner were centrifuged, re-suspended in the lysis buffer described in Table 1 and homogenized with a BRAUN MUSK mechanical homogenizer using glass balls of 0.45 mm diameter.

The cell extract obtained was ultracentrifuged at 80,000 rpm for 30 minutes at 4°C to separate the cell membranes from the rest of the extract.

The cell membranes were re-suspended in the TNE buffer described in Table 1, aliquoted and frozen to -80°C. The protein concentration was determined by Bradford colorimetric microdosing, using bovine gammaglobulin as reference, as described by Bradford H.B. (1976): "Anal. Biochem.", vol 72, pp 248-254.

The membranes were used to verify the presence of the active protein β₂AR using the method described by Lefkowitz et al. (1990): "Biochemistry", vol 29, pp 2335-2342.

This method consists of identifying the binding affinity of the adrenergic protein present in the membrane preparation with the antagonist cyanopindolol labelled with radioactive iodine.

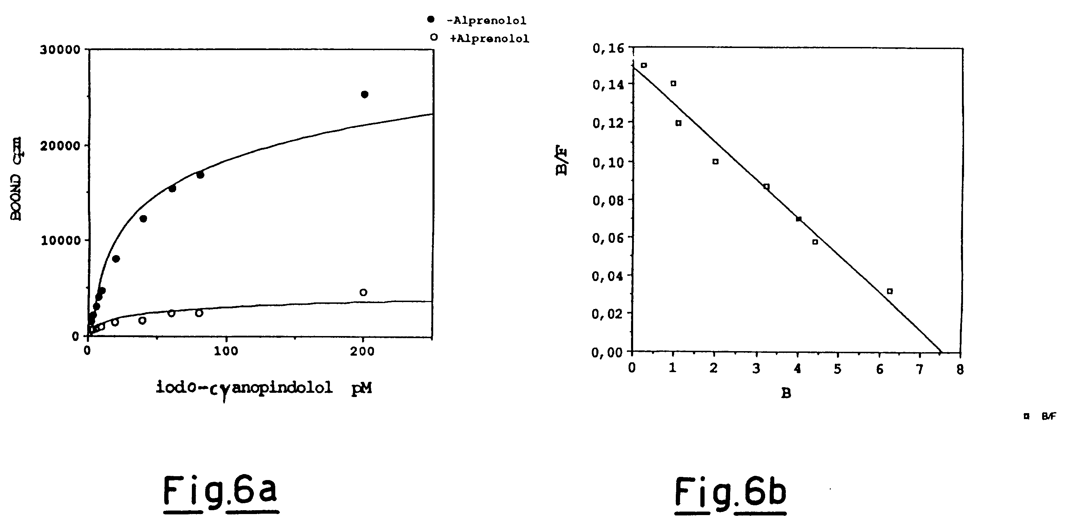

For this purpose, 5 µg of pEV-STE2-β₂AR yeast transformer membrane protein, prepared as heretofore described, were added to 1 ml of TNE buffer (Table 1) containing increasing concentrations, from 4 pM to 200 pM, of labelled (¹²⁵I)-CYP iodo-cyanopindolol (2000 Ci/mmole, from Amersham, UK) in the absence (curve ●) and in the presence (curve o) of the alprenolol competitor at a final concentration of 10 µM. The proteins were incubated at 37°C for one hour and filtered through GF/C (Whatman) filters. The filters were washed four times with 4 ml of TNE solution (Tab. 1). The values in cpm of the radioactivity retained in each filter, measured with a BECKMANN gamma-counter, are shown in graph a) of Figure 6. The indicated values represent the mean of three independent experiments. The cells of the untransformed strain do not exhibit activity.

Using Scatchard analysis as described by Schatchard G. (1949): "Ann. N.Y. Acad. Sci.", vol 51, pp 660-672, it was calculated that the K_{D} of the receptor is 20 pM and the Bₘₐₓ (ie the maximum quantity of iodocyanopindolol bound specifically to the receptor under saturation conditions) is 0.75 pmoles/mg of protein (Figure 6b).

The binding affinity of the protein STE-β₂AR in the transformant was analogous to that observed in preceding works regarding the expression of the human receptor β₂AR in mammal cells as described by Dohlman H.G. et al. (1990): "Biochemistry", vol 29, page 2335.

The inhibiting effect of certain chemical substances on the binding efficiency of the chimeric protein STE2-β₂AR with (¹²⁵I)-iodo-cyanopindolol was also analyzed.

The object was to verify whether the inhibiting efficiency of the antagonist alprenolol and of the agonists isoproterenol and epinephrine was the same as observed in analogous experiments conducted on human cells.

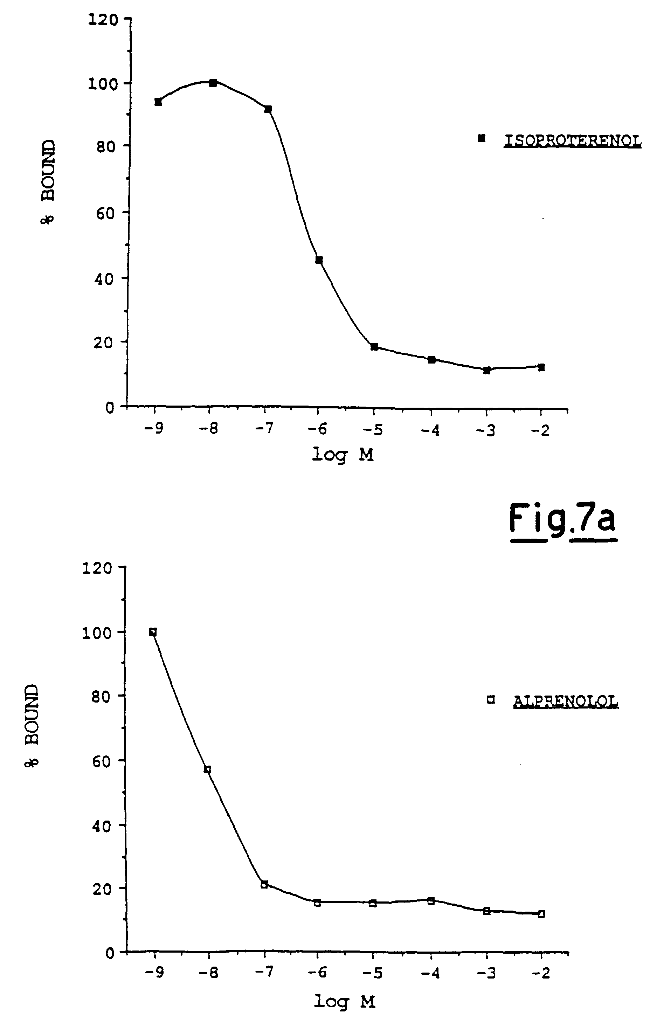

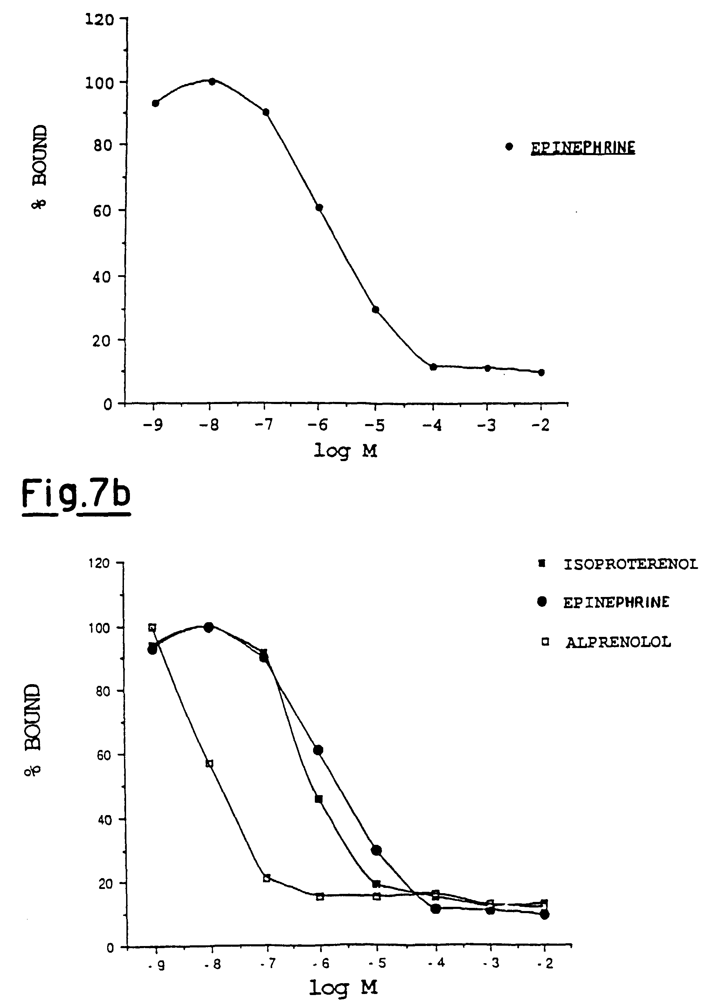

The data obtained from the inhibition experiments are reported in Figures 7a and 7b.

The yeast membrane proteins were incubated at 37°C for one hour in 1 ml of TNE solution (Table 1) containing (¹²⁵I)-CYP at a concentration of 20 pM and increasing concentrations of each competitor from 10⁻⁹ M to 10⁻³ M.

The three substances inhibit the bond with (¹²⁵I)-CYP in a different manner and follow the inhibition efficiency order alprenolol > isoproterenol > epinephrine.

The aforedescribed experiment confirms that the human β₂-adrenergic receptor protein totally preserves the binding capacity already observed in other systems, as described by Lefkowitz R.J. et al. (1988): "Scienze", vol 240, pp 1310-1316.

A test of the binding efficiency with (¹²⁵I)-CYP by immobilizing the membrane proteins of Schizosaccharomyces pombe transformants on a filter.

For this purpose increasing quantities, 5, 15 and 20 µg, of membrane proteins prepared from Schizosaccharomyces pombe transformants with the plasmids pEVP11, pEV-STE2-β₂AR and pEV-β₂AR were fixed onto nitrocellulose filters. The filters were treated to analyze the bond between the β₂AR receptor and (¹²⁵I)-iodo-cyanopindolol by the method described by GalIi et al. (1990): "Biochem. Biophys. Res. Comm.", vol 173, pp 680-688.

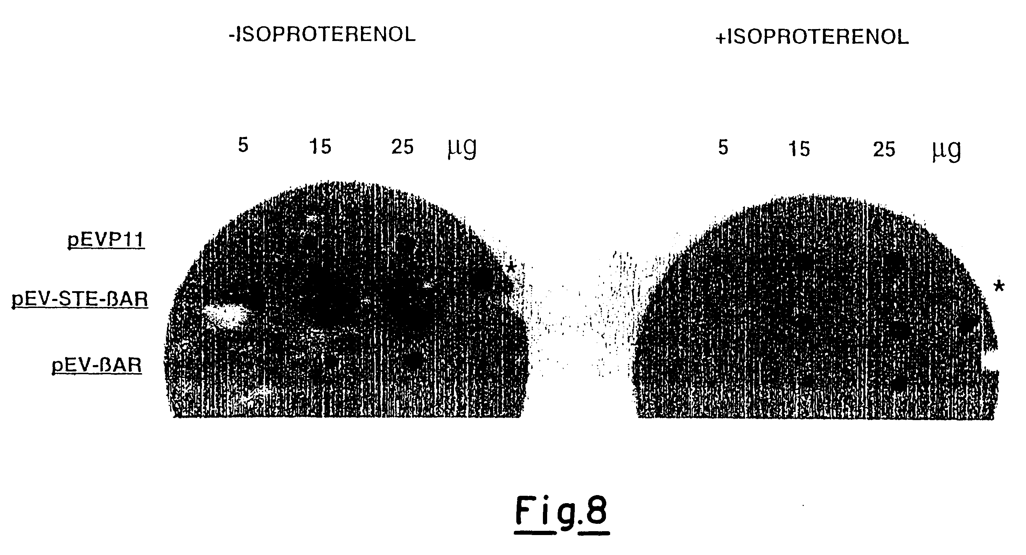

The filters were incubated for 60 minutes in 10 ml of TNE (Table 1) containing 20 mg/ml of bovine serum albumen and were then transferred for 90 minutes into 10 ml of TNE (Table 1) containing (¹²⁵I)-CYP (20 pM) and (¹²⁵I)-CYP (20 pM) plus isoproterenol (10 µM) ( 8). The filters were washed four times for 15 minutes in 10 ml of TNE (table 1) containing 0.2% Tween 20, dried and exposed on an autoradiographic plate at -70°C. Figure 8 shows the results obtained.

The most intense autoradiographic signals correspond to the spots relative to membrane proteins of the transformant pEV-STE2-β₂AR.

This signal disappears in the presence of the inhibitor isoproterenol.

With the membrane proteins of the transformant pEV-β₂AR a low autoradiographic signal is observed, explained as a lesser quantity of human receptor molecule per cell. Again in this case the signal decreases drastically in the presence of isoproterenol The spots indicated by the asterisk * indicate the membrane proteins extracted from pig brain tissue where the β₂-adrenergic receptor is present in a high concentration and used as control.

It can therefore be concluded that the binding properties of the β₂-adrenergic receptor expressed in the yeast Schizosaccharomyces pombe remain unaltered when the protein is immobilized on a cellulose filter.

**TABLE 1**

| | |
|---|---|
| SCE | 1 M Sorbitol |
| | 0.1 M Sodium citrate |
| | 0.06 M EDTA pH 7 |
| LYSIS BUFFER | 5 mM Tris-HCl pH 7.4 |
| | 5 mM EDTA |
| TNE | 50 mM Tris-HCl pH 7.4 |
| | 150 mM NaCl |
| | 5 mM EDTA |

## Claims

1. Method for preparing the human β₂-adrenergic receptor, comprising the steps of:
(a) transforming Schizosaccharomyces pombe cells with a vector p-EV-STE2-β₂AR as disclosed in Figure 4 and
(b) growing in suitable conditions the so transformed cells.

2. A modified cloning vector (YIP5-oligo) containing an oligo with BamHI-AatII ends of 64 nucleotide bases corresponding to the 11 non-encoding bases and the first 42 encoding bases of the gene STE2 of the yeast Saccharomyces cerevisiae, cloned in the vector YIP5, as disclosed in Figure 1.

3. A expression vector (pEM-β₂AR) containing a restriction fragment NcoI-PvuII comprising the gene β₂AR cloned on the site SmaI of the vector pEMBLyex2, as disclosed in Figure 2.

4. A modified cloning vector (YI-STE2-β₂AR) containing a biologically active hybrid of the adrenergic receptor consisting of a deletion of the first 23 amino acids of the amino-terminal end, these being replaced by the encoding Sequence for the first 14 amino acids of the gene STE2 of Saccharomyces cerevisiae, as disclosed in Figure 3.

5. A modified vector (pEV-STE2-β₂AR) for expressing the chimeric gene STE2-β₂AR of claim 4 in Schizosaccharomyces pombe, as disclosed in Figure 4.

6. A modified vector (pEV-β₂AR) for expression in Schizosaccharomyces pombe containing the restriction fragment SstI-BamHI comprising the sequence encoding the entire adrenergic receptor cloned in the vector pEVP11, as disclosed in Figure 5.

7. Transformant of Schizosaccharomyces pombe containing the modified vector of claim 5.

8. Transformant in accordance with claim 7, wherein the transformed cells derive from the yeast strain Schizosaccharomyces pombe leul-32 h⁻.

9. A method for verifying the presence of the biologically active receptor by exposing cultures of Schizosaccharomyces pombe transformed with the plasmids of claim 5 or 6, to different agonistic and antagonistic ligands.

10. A method for screening ligands characterised by immobilizing membrane proteins of Schizosaccharomyces pombe transformants containing the plasmids of claim 5 or 6, and testing the binding activity of ligands.

## Patentansprüche

1. Verfahren zur Herstellung des menschlichen β₂-adrenergischen Rezeptors, das die Schritte aufweist:
(a) transformieren von Schizosaccharomyces pombe Zellen mit einem Vektor p-EV-STE2-β₂AR, wie in Figur 4 offenbart, und
(b) vermehren der so transformierten Zellen unter geeigneten Bedingungen.

2. Modifizierter Kloniervektor (YIP5-oligo), der ein Oligo enthält mit BamHI-AatII Enden von 64 Nucleotidbasen, die den 11 nicht-kodierenden Basen und den ersten 42 kodierenden Basen des Gens STE2 der Hefe Saccharomyces cerevisiae entsprechen, kloniert in dem Vektor YIP5, wie in Figur 1 offenbart.

3. Expressionsvektor (pEM-β₂AR), der ein Restriktionsfragment NcoI-PvuII enthält, das das an der Stelle SmaI des Vektors pEMBLyex2 klonierte Gen β₂AR aufweist, wie in Figur 2 offenbart.

4. Modifizierter Kloniervektor (YI-STE2-β₂AR), der ein biologisch aktives Hybrid des adrenergischen Rezeptors enthält, der aus einer Deletion der ersten 23 Aminosäuren des Amino-Terminus besteht, wobei diese durch eine kodierende Sequenz für die ersten 14 Aminosäuren des Gens STE2 von Saccharomyces cerevisiae ersetzt sind, wie in Figur 3 offenbart.

5. Modifizierter Vektor (pEV-STE2-β₂AR) für die Expression des chimären Gens STE2-β₂AR von Anspruch 4 in Schizosaccharomyces pombe, wie in Figur 4 offenbart.

6. Modifizierter Vektor (peV-β₂AR) für die Expression in Schizosaccharomyces pombe, der das Restriktionsfragment SstI-BamHI mit der Sequenz enthält, die den gesamten adrenergischen Rezeptor, kloniert in dem Vektor pEVP11, kodiert, wie in Figur 5 gezeigt.

7. Transformand von Schizosaccharomyces pombe, der den modifizierten Vektor von Anspruch 5 enthält.

8. Transformand nach Anspruch 7, bei dem die transformierten Zellen von dem Hefestamm Schizosaccharomyces pombe leul-32 h⁻ stammen.

9. Verfahren zum Überprüfen der Anwesenheit des biologisch aktiven Rezeptors durch Aussetzen der Kulturen von Schizosaccharomyces pombe, die mit den Plasmiden der Ansprüche 5 oder 6 transformiert sind, verschiedenen agonistischen und antagonistischen Liganden.

10. Verfahren zum Screening von Liganden, dadurch gekennzeichnet, daß Membranproteine von Schizosaccharomyces pombe Transformanden, die Plasmide der Ansprüche 5 oder 6 enthalten, immobilisiert werden und die Bindungsaktivität der Liganden geprüft wird.

## Revendications

1. Procédé de préparation du récepteur β₂-adrénergique humain, comprenant les étapes consistant à :
(a) transformer des cellules de Schizosaccharomyces pombe par un vecteur p-EV-STE2-β₂AR tel que représenté à la figure 4 et
(b) cultiver les cellules ainsi transformées dans des conditions appropriées.

2. Vecteur de clonage modifié (YIP5-oligo) contenant un oligonucléotide ayant des extrémités BamHI-AatII de 64 bases nucléotidiques correspondant aux 11 bases non codantes et au 42 premières bases codantes du gène STE2 de la levure Saccharomyces cerevisiae, cloné dans le vecteur YIP5, comme représenté à la figure 1.

3. Vecteur d'expression (pEM-β₂AR) contenant un fragment de restriction NcoI-PvuII comprenant le gène β₂AR cloné sur le site SmaI du vecteur pEMBLyex2, comme représenté à la figure 2.

4. Vecteur de clonage modifié (YI-STE2-β₂AR) contenant un hybride bioactif du récepteur adrénergique constitué par une délétion des 23 premiers acides aminés de l'extrémité amine terminale, ceux-ci étant remplacés par la séquence codante des 14 premiers acides aminés du gêne STE2 de Saccharomyces cerevisae, comme représenté à la figure 3.

5. Vecteur modifié (pEV-STE2-β₂AR) destiné à l'expression du gène chimère STE2-β₂AR selon la revendication 4 dans Schizosaccharomyces pombe, comme représenté à la figure 4.

6. Vecteur modifié (pEV-β₂AR) destiné à l'expression dans Schizosaccharomyces pombe contenant le fragment de restriction SstI-BamHI comprenant la séquence codant le récepteur adrénergique entier cloné dans le vecteur pEVP11, comme représenté à la figure 5.

7. Transformant de Schizosaccharomyces pombe contenant le vecteur modifié selon la revendication 5.

8. Transformant selon la revendication 7, dans lequel les cellules transformées proviennent de la souche de levure Schizosaccharomyces pombe leul-32h⁻.

9. Procédé de vérification de la présence du récepteur bioactif par exposition de cultures de Schizosaccharomyces pombe transformées par les plasmides selon les revendications 5 ou 6 à différents ligands agonistes et antagonistes.

10. Procédé de criblage de ligands, caractérisé par l'immobilisation de protéines membranaires de transformants de Schizosaccharomyces pombe contenant les plasmides selon la revendication 5 ou 6 et l'essai de l'activité de fixation des ligands.
